# FASCICULE DE BREVET EUROPEEN

(11) **EP 3 335 039 B1**
(45) Date de publication et mention de la délivrance du brevet: **06.03.2024**
(21) Numéro de dépôt: 16758265.9
(22) Date de dépôt: 10.08.2016
(51) Int. Cl.: G01N 33/00, G04G 21/02

(54) **DISPOSITIF DE MESURE DE GAZ INCORPORÉ DANS UNE MONTRE**
IN EINE UHR INTEGRIERTE GASMESSVORRICHTUNG
GAS MEASUREMENT DEVICE INCORPORATED INTO A WATCH

(30) Priorité: 13.08.2015 CH 11602015
(43) Date de publication de la demande: 20.06.2018
(73) Titulaire: Take Off Diffusion S.A., 2520 La Neuveville (CH)
(72) Inventeur: VOUMARD, Olivier, 2525 Le Landeron (CH)
(74) Mandataire: Cronin, Brian Harold John
(86) Numéro de dépôt international: PCT/IB2016/001104
(87) Numéro de publication internationale: WO 2017/025791

(56) Documents cités:
- EP-A2- 1 293 946
- GB-A- 2 331 363
- US-A1- 2010 074 059
- US-A1- 2011 034 792

## Description

La présente invention se rapporte aux dispositifs de mesure de gaz.

Les dangers dus aux gaz peuvent avoir différentes origines comme par exemple une fuite d'un ou plusieurs gaz toxiques, une déficience ou une trop forte concentration d'oxygène, voire la présence de gaz inflammables. S'il n'est pas détecté de manière précoce, le personnel, les lieux et les équipements ne peuvent être protégés.

En fonction du besoin, il peut être nécessaire de choisir un détecteur très spécifique ou au contraire capable de détecter une grande variété de gaz.

EP 1 293 946 décrit une montre agencée pour signaler la concentration d'un gaz comportant deux états de fonctionnement, un état « heure » et un état « alarme ». Dans l'état « heure » la concentration de gaz et l'heure sont affichés simultanément ou successivement.

Les principaux risques qui découlent de la présence de gaz sont une explosion due à la présence de gaz inflammables et une intoxication ou une asphyxie due à la présence de gaz toxiques.

Un détecteur de gaz peut donc se révéler nécessaire en tant que protection individuelle pour des activités exposées à un risque. Des détecteurs de gaz portables peuvent répondre à des besoins professionnels ponctuels, ou bien, dans beaucoup d'applications industrielles, la sécurité doit être assurée continuellement par un système de détection de gaz à poste fixe.

Le besoin de mesurer en même temps et en continu la concentration de plusieurs gaz est de plus en plus fréquent dans les applications industrielles (industries chimiques et agro-alimentaires mais aussi chaufferies, tunnels ...)

Les gaz toxiques proviennent de sources diverses, comme par exemple la combustion (CO-NO-NO2-SO2), l'incinération (H2S-NH3-HCI) ou la fermentation (H2S-NH3).

Leur présence, passive ou active, en milieu industriel est fréquente (chimie, pétrochimie, chaînes de froid, agroalimentaire) et rend leur détection indispensable. Les risques d'intoxication sont liés au temps d'exposition d'un sujet à un produit néfaste. Le danger s'apprécie en valeur moyenne d'exposition et en valeur limite d'exposition. Ces valeurs s'expriment à la fois en poids (mg/m3) et en volume (partie par million, ppm).

Les détecteurs de gaz portable permettent de savoir si l'atmosphère ambiante est explosive ou non, ce qui est fondamental pour la sécurité des premiers intervenants sur un incendie par exemple. Le danger d'explosion provoqué par tel ou tel gaz est bien souvent sous-estimé et l'intervenant doit compter sur un appareillage de détection et de mesure de l'explosivité, peu encombrant, fiable, et de mise en oeuvre facile.

Le principal inconvénient de ces appareils portables est qu'ils doivent être portés dans un lieu à risque et qu'ils sont très souvent déposés dès que la zone à risque est quittée. Dès lors, une personne peut se retrouver dans une zone présumée non à risque, comme par exemple son domicile, et être exposée à la présence d'un gaz toxique comme le monoxyde de carbone.

Le but de la présente invention est de proposer une alternative à tous ces appareils, et plus particulièrement, de proposer une montre-bracelet comportant un détecteur de gaz qui permet de mesurer la concentration de gaz dans les zones à risques, comme dans les zones que l'on pense être sans risques.

Conformément à l'invention, un dispositif pour mesurer la concentration d'un gaz composant l'air ambiant comporte une montre-bracelet comprenant une boite de montre contenant un mouvement horloger, des organes indicateurs de l'heure visibles à travers une glace de la boite de montre. En outre, la montre-bracelet comprend au moins un capteur agencé pour mesurer la concentration d'un gaz composant de l'air ambiant, ce capteur étant situé dans un logement de la boite de montre ou d'un élément rapporté à la boite de montre et présentant une surface à l'intérieur de ce logement exposé à l'air ambiant à travers au moins un orifice, l'orifice se trouvant sur une partie de la boite de montre ou sur l'élément rapporté qui n'est pas en contact avec le poignet du porteur lorsque la montre-bracelet est porté au poignet. La montre-bracelet comporte également un organe indicateur de la valeur mesurée de la concentration de gaz, cet organe indicateur permettant de connaître la valeur de la concentration de gaz mesurée instantanément. Il comporte aussi des moyens pour actionner une alerte. Le dispositif comporte deux états de fonctionnement, un état « heure » et un état « alarme ».

Selon l'invention, le dispositif est configuré pour fonctionner selon une séquence opérationnelle complète comprenant les trois étapes suivantes a), b) et c) :
a) En l'état « heure » les organes indicateurs de l'heure indiquent l'heure et le capteur mesure en permanence la concentration de gaz sans l'afficher et sans actionner une alerte en l'absence d'une concentration mesurée de gaz ;
b) Au cas où le capteur détecte une concentration d'un gaz lorsque le capteur est dans l'état « heure », le dispositif bascule automatiquement en l'état « alerte » dans lequel les organes indicateurs de l'heure sont activés pour indiquer l'heure ou inactivés pour ne pas indiquer l'heure, et dans lequel l'organe indicateur de la valeur mesurée de la concentration de gaz affiche cette valeur mesurée et en outre les moyens pour actionner une alerte actionnent une alerte lors de l'atteinte ou du franchissement d'un seuil de la concentration de gaz mesurée par le capteur ; et
c) Au cas où le capteur ne détecte plus une concentration d'un gaz lorsqu'il est dans l'état « alerte », le dispositif bascule automatiquement en l'état « heure » où les organes indicateurs de l'heure indiquent l'heure et le capteur mesure en permanence la concentration de gaz sans l'afficher et sans déclencher d'alerte en l'absence de concentration de gaz mesurée ; et dans lequel la montre-bracelet comprend en outre un moyen de communication sans fil pour transmettre des informations à un autre appareil, à savoir le moyen de communication sans fil est agencé pour transmettre à un smartphone ou à un ordinateur, des informations indiquant que ladite valeur seuil de la concentration de gaz mesurée dans l'air ambiant a été atteinte ou dépassée.

Dans une forme d'exécution, l'organe indicateur de la valeur mesurée de la concentration de gaz peut également indiquer la valeur maximale de la concentration de gaz mesurée.

Dans une variante, la montre-bracelet peut comporter un capteur pour mesurer la valeur de la concentration de gaz mesurée instantanément et des moyens pour actionner une alerte lors de l'atteinte ou du franchissement d'un seuil de la concentration de gaz mesurée par le capteur sans que le porteur de la montre-bracelet ne connaisse la concentration atteinte.

Dans une forme d'exécution préférentielle, la montre comporte des moyens d'alerte comme par exemple une alarme lumineuse et/ou une alarme sonore et/ou une alarme vibrante.

Dans une forme d'exécution, la montre-bracelet comporte une unité de contrôle agencée pour mesurer l'état de fonctionnement et/ou la durée de vie de composants intégrés dans la boite de montre, notamment le capteur ou la durée de vie d'une source d'alimentation.

Dans une forme d'exécution, la montre-bracelet peut comporter une unité de stockage agencée pour conserver un historique des valeurs mesurées de la concentration de gaz et optionnellement les mesures des composants intégrés dans la boite de montre et inspectés par une unité de contrôle. Par exemple, la montre peut enregistrer dans cette unité de stockage un historique des concentrations, des durées d'exposition, des profils de concentration de gaz pendant une exposition, un maximum de concentration pendant une exposition, un nombre d'expositions, une durée de vie des sources d'alimentation, une durée de vie du capteur. Toutes ces informations peuvent être accessibles à l'utilisateur soit directement sur un affichage de la montre, soit par un appareil déporté comme par exemple un smartphone.

Selon cette forme d'exécution, les moyens d'actionnement d'une alerte peuvent être enclenchés lorsque l'unité de contrôle indique qu'au moins un composant intégré dans la boite de montre nécessite une intervention extérieure.

La montre-bracelet peut comporter des aiguilles pour l'affichage de l'heure et les mêmes aiguilles ou d'autres aiguilles pour l'affichage de la valeur mesurée de la concentration de gaz.

Dans une variante, la montre-bracelet peut comporter un affichage digital pour l'affichage de l'heure et le même affichage digital ou un autre affichage digital pour l'affichage de la valeur mesurée de la concentration de gaz.

Dans une version combinée d'affichage, la montre-bracelet peut comporter des aiguilles pour l'affichage de l'heure et un affichage digital pour afficher la valeur mesurée de la concentration de gaz mesurée et vice et versa.

Pour en faciliter la maintenance, la montre-bracelet peut comporter une unité de traitement apte à recevoir des informations d'un appareil externe par exemple un smartphone ou un PC pour configurer la montre, notamment pour étalonner le capteur ou pour une mise à jour de l'heure.

Dans une forme d'exécution, la montre-bracelet peut comporter, à l'intérieur de la boite de montre, un émetteur pour transmettre des informations et un récepteur pour recevoir des informations. Ainsi, une communication sans fil peut permettre notamment de gérer les configurations de la montre, de transmettre des alarmes, de transférer des données internes de la montre comme une initialisation et un réglage de la montre, une mise à l'heure de la montre. D'autres fonctions, comme une transmission des alarmes, un transfert des données en mémoires, ou un étalonnage et une calibration d'un capteur de gaz peuvent être effectuées.

En fonction des besoins, la montre peut comporter une pluralité de capteurs agencés pour mesurer respectivement la concentration d'un gaz particulier composant de l'air ambiant. La montre peut être équipée de capteurs pour détecter par exemple le sulfure d'hydrogène, ou le monoxyde de carbone.

En outre, le dispositif inventif comporte facultativement un état « sommeil » dans lequel le capteur est désactivé, et au moins un bouton poussoir, actionnable lorsque le dispositif en dans l'état « sommeil » pour activer le dispositif en état « heure ».

Les moyens pour actionner une alerte peuvent actionner une alerte à plusieurs niveaux selon la concentration de gaz mesurée par le capteur, chaque niveau d'alerte correspondant à un ou plusieurs bips sonores ou lumineuse ou des vibrations, dont l'intensité et la fréquence peuvent varier.

Dans certaines exécutions, les organes indicateurs de l'heure comporte un cadran et au moins deux aiguilles de longueurs différentes pivotantes autour d'un axe, le dispositif comprenant en outre un segment du cadran s'étendant au plus sur 150° et de préférence au plus sur 120°, ce segment de cadran portant vers sa périphérie au moins deux zones arquées avec des graduations ou autres indications concernant une concentration de gaz mesurée et/ou une opération de maintenance du dispositif, ces zones étant décalées radialement. Dans cette configuration, les aiguilles coopèrent avec le cadran pour indiquer l'heure lors de l'état « heure », et les aiguilles de longueur différente coopèrent avec les zones arquées décalées radialement lors de l'état « alerte » pour donner au moins une indication relative à la concentration de gaz mesurée et facultativement une indication de maintenance du dispositif.

Selon une exécution, les organes indicateurs de l'heure comportent un cadran, le dispositif comprenant en outre un disque rotatif dont une partie de la circonférence est visible dans un guichet du cadran. La circonférence du disque porte une série d'indications relative à la concentration du gaz mesurée, le fonctionnement du dispositif et/ou la durée de vie de composants intégrés dans la boîte de montre et/ou la durée de vie d'une source d'alimentation. Le dispositif comporte en outre des moyens pour faire tourner le disque afin de faire venir une indication choisie dans le guichet et, selon l'indication choisie, pour faire fonctionner le dispositif où pour permettre de calculer différentes paramètres de concentration de gaz ou faire les opérations nécessaires pour remettre le dispositif en état de fonctionnement.

Les caractéristiques de l'invention apparaîtront plus clairement à la lecture de la description de plusieurs formes d'exécutions données uniquement à titre d'exemple, nullement limitative en se référant aux figures schématiques, dans lesquelles :
- La figure 1 représente une vue de face d'une montre bracelet comportant un affichage à aiguilles pour l'indication de l'heure, un orifice, pour laisser passer le gaz vers le capteur, disposé sur un élément rapporté à la boite de montre, dans lequel se trouve un capteur de gaz pour mesurer la concentration de gaz dont la valeur est donnée sur une échelle indicatrice dédiée à l'aide d'une des aiguilles ;
- La figure 2 représente une vue de face d'une montre bracelet comportant un affichage à aiguilles pour l'affichage de l'heure, un orifice, pour laisser passer le gaz vers le capteur de gaz, disposé sur une corne de la boite de montre, dans laquelle se trouve un capteur de gaz pour mesurer la concentration de gaz dont la valeur est donnée sur un affichage déporté à l'aide d'une autre aiguille;
- La figure 3 représente une vue de face d'une montre bracelet comportant un affichage digital pour l'affichage de l'heure, un orifice disposé sur la bande de carrure pour laisser passer le gaz vers le capteur de gaz pour mesurer la concentration de gaz dont la valeur de la concentration de gaz mesurée s'affiche également sur l'affichage digital ;

- La figure 4 représente une vue de face d'une montre bracelet comportant un affichage digital pour l'affichage de l'heure, un orifice disposé sur la bande de carrure pour laisser passer le gaz vers le capteur de gaz pour mesurer la concentration de gaz dont la valeur de la concentration de gaz mesurée s'affiche sur un autre affichage digital ;
- La figure 5 représente une vue de face d'une montre bracelet comportant un affichage à aiguilles pour l'affichage de l'heure, un orifice disposé pour laisser passer le gaz vers le capteur de gaz pour mesurer la concentration de gaz dont la valeur de la concentration de gaz mesurée s'affiche sur un affichage digital ;
- La figure 6 représente une vue en perspective d'une montre bracelet comportant un affichage à aiguilles pour l'affichage de l'heure, un orifice disposé sur la bande de carrure de la boite de montre pour laisser passer le gaz vers le capteur de gaz pour mesurer la concentration de gaz dont la valeur de la concentration de gaz mesurée s'affiche sur un affichage digital ;
- La figure 7 représente l'intérieur d'une boite de montre dont seuls le logement dans lequel est disposé le capteur, le capteur lui-même et un microprocesseur relié audit capteur sont illustrés ;
- La figure 8 représente une vue de face d'une montre bracelet selon la présente invention ;
- La figure 9 représente une vue éclatée partielle de la montre bracelet de la figure 8 ;
- La figure 10 représente une vue de face d'un cadran de montre de la montre bracelet de la figure 8 ; et
- La figure 11 représente un disque de la montre bracelet de la figure 8 ; et
- La figure 12 représente une vue de face d'une montre bracelet selon la présente invention.

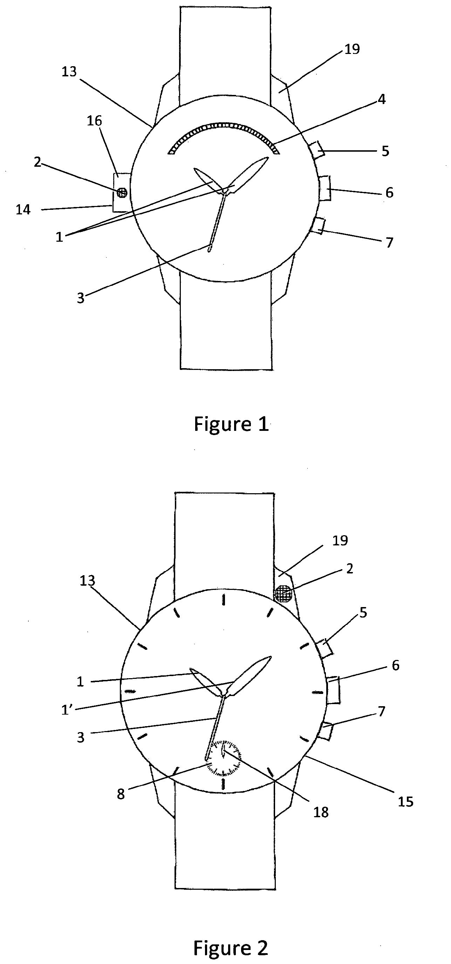

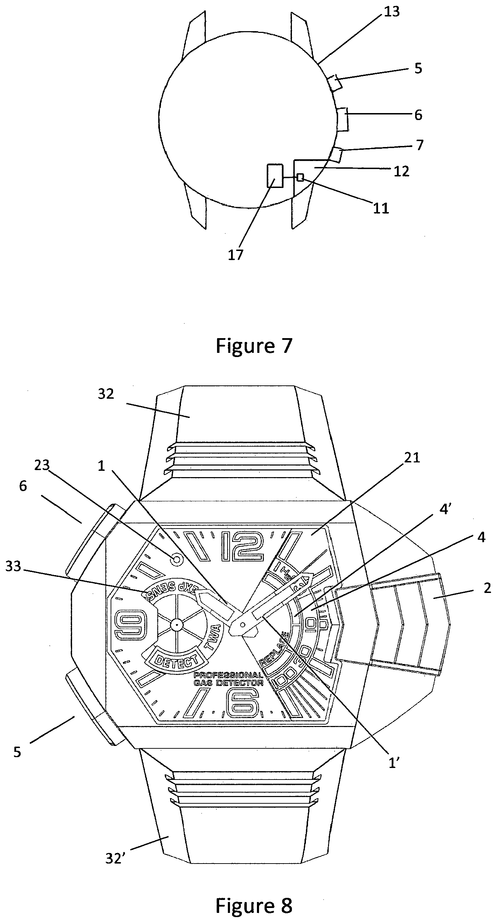

Comme illustrés aux figures 1 à 7, une montre bracelet mesure la présence de gaz dans l'air ambiant à l'aide d'un capteur 11 disposé, derrière un orifice 2, à l'intérieur de la boite de montre 13 (figure 7) ou sur un élément rapporté 14 (figure 1) ou dans une corne 19 de la boite de montre 13 (figure 2).

Comme illustré à la figure 1, la montre, comportant une boite de montre 13, et des cornes 19 pour fixer un bracelet, affiche l'heure de manière conventionnelle avec des aiguilles heures et minutes 1, 1' et une aiguille des secondes 3. Le capteur détecte le gaz à travers un orifice 2 disposé sur un élément rapporté 14 de la boite de montre 13 à 9H. L'orifice 2 se trouve sur une face extérieure 16 de l'élément rapporté 14, l'orifice 2 n'étant pas en contact avec le poignet du porteur lorsque la montre-bracelet est portée. Une échelle graduée 4 indique la valeur de la concentration de gaz mesurée. La montre comporte deux boutons poussoirs 5, 7 et une couronne de remontoir 6 pour la mise à l'heure. Lorsque l'utilisateur appuie sur un des boutons poussoir 5, 7, une information est transmise à un microprocesseur 17 (figure 7) relié électriquement au capteur 11, lui indiquant de mesurer la concentration de gaz instantanée. Dès lors, l'aiguille des secondes 3 indique sur l'échelle graduée 4 la valeur instantanée de la concentration de gaz mesurée. Lorsque l'utilisateur appuie une deuxième fois sur le même bouton poussoir 5, 7 l'aiguille des secondes 3 reprend sa fonction de base, à savoir indication du temps. Lorsque le second bouton poussoir est activé 5, 7, l'aiguille des secondes 3 indique par exemple la valeur maximale de la concentration de gaz mesurée lors des quatre dernières heures. Cette information est stockée dans le microprocesseur 17 et reste accessible à la demande. Lorsque le seuil de la concentration de gaz mesurée a été atteint ou dépassé, le capteur de gaz 11 envoie un signal électrique au microprocesseur 17 pour enclencher des moyens d'alerte.

Les moyens d'alerte peuvent par exemple être une alarme lumineuse, une alarme sonore, une alarme vibrante, ou une alerte transmise sans fil à un appareil déporté, par exemple smartphone, voire à un réseau informatique.

Comme illustré à la figure 2, la montre affiche l'heure de manière conventionnelle avec des aiguilles heures et minutes 1 et une aiguille des secondes 3. Le capteur détecte le gaz à travers un orifice 2 disposé sur une corne 19 de la boite de montre 13. Une échelle graduée 8 à 6H permet de déterminer la valeur de la concentration de gaz mesurée à l'aide d'une aiguille dédiée 18. Lorsque le seuil de la concentration de gaz mesurée a été atteint ou dépassé les moyens d'alerte sont enclenchés.

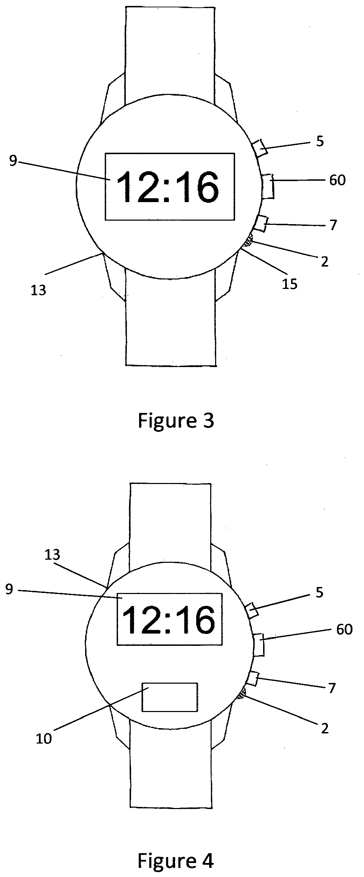

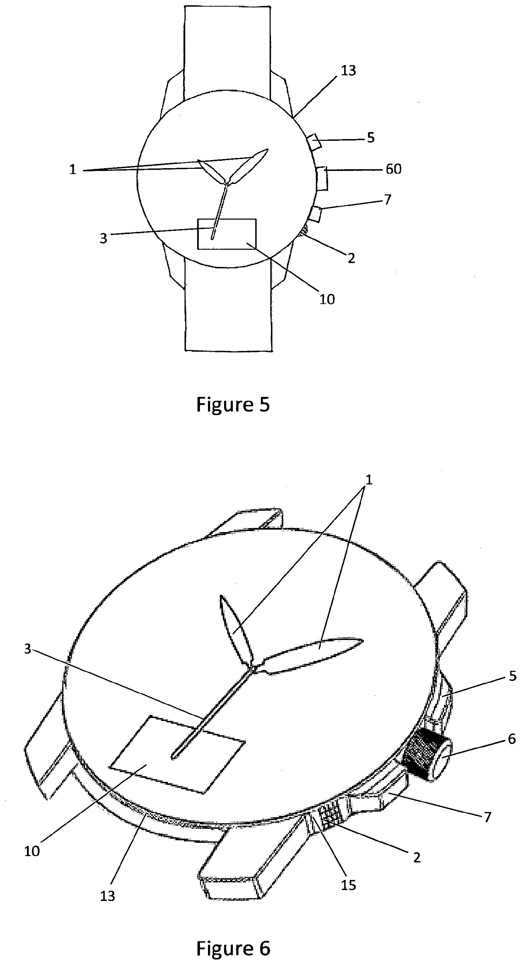

Comme illustré à la figure 3, la montre affiche l'heure à l'aide d'un affichage digital 9. Le capteur détecte le gaz à travers un orifice 2 disposé sur la bande carrure 15 de la boite de montre 13. La vue en perspective illustrée à la figure 6, permet de voir l'orifice 2 sur la bande carrure 15. La montre comporte trois boutons poussoirs 5, 60, 7. Lorsque l'utilisateur appuie sur un des boutons poussoirs 5, 60, 7, l'affichage digital 9 bascule sur l'indication de la valeur de la concentration gaz mesurée. Lorsque l'utilisateur appuie sur le même bouton poussoir 5, 60, 7, l'information de l'indication horaire apparaît à nouveau sur l'affichage digital 9. Lorsque le seuil de la concentration de gaz mesurée a été atteint ou dépassé, les moyens d'alerte sont enclenchés et l'affichage digital 9 indique la concentration de gaz mesurée.

La montre illustrée à la figure 4, affiche l'heure de manière digitale sur un premier afficheur 9 et la valeur de la concentration de gaz de manière digitale également sur un autre afficheur digital 10. La montre comporte également trois boutons poussoirs 5, 60, 7 pour les réglages de la montre et pour accéder aux fonctions annexes. Dans l'exemple illustré à la figure 5, la montre affiche l'heure avec des aiguilles heures et minutes 1 et une aiguille des secondes 3 et la valeur de la concentration de gaz de manière digitale est indiquée sur un afficheur digital 10.

La montre illustrée à la figure 6, comporte un affichage à aiguille pour l'indication de l'heure et un affichage digital pour les fonctions de la mesure de la concentration de gaz. Parmi ces fonctions, la montre enregistre dans une mémoire interne, par exemple du microprocesseur 17 (figure 7), l'historique des concentrations de durées d'exposition, de profil de concentration de gaz pendant une exposition, de maximum de concentration pendant une exposition, le nombre d'expositions, la durée de vie des batteries, la durée de vie du capteur, durée de vie des consommables.

Les informations que l'expéditeur envoie à un destinataire doivent nécessairement transiter à travers un support de transmission. L'espace libre est utilisé comme support de transmission et dans ce cas, il est question de transmission sans fil. La montre est donc munie d'un système de communication sans fil. La communication sans fil permet de gérer les configurations de la montre, de transmettre des alarmes et de transférer les données internes comme l'initialisation et le réglage de la montre, la mise à l'heure de la montre, le mode de transmission des alarmes, le transfert des données en mémoires, la calibration du ou des capteurs de gaz.

La transmission sans fil s'appuie sur la propagation des ondes électromagnétiques ou ondes hertziennes ou encore des ondes radioélectriques. Le concept radio indique toute communication assurée sans support matériel, ainsi le terme radiocommunication englobe toutes les communications sans fil. La communication sans fil peut par exemple être assurée par un satellite de communication, par des communications mobiles cellulaires, par wi-fi, par Bluetooth ou par RFID.

Dans le cas de satellite de télécommunications, un satellite se sert des ondes électromagnétiques ou hertziennes pour relier deux points terrestres, soit le point où est situé le porteur de la montre et un point où est localisé un poste de surveillance. Les satellites de télécommunications sont utilisés pour toutes sortes de communications: radiodiffusion sonore, télévision, téléphonie, transmission de données, etc.

Dans le cas de l'emploi de Wi -Fi, le porteur de la montre devra se trouver à proximité d'un routeur qui assure la liaison et qui fonctionne comme un hub de communication sans fil.

Dans le cas de l'emploi du Bluetooth, protocole qui établit une connexion sans fil sécurisée de courte portée entre deux dispositifs, le porteur de la montre devra se trouver à une distance encore plus proche de l'appareil auquel doit être transmis un signal. Le Bluetooth est similaire au Wi -Fi, mais la faible puissance utilisée minimise la portée et le débit binaire disponible est généralement bas.

Dans le cas de l'emploi de puce RFID, qui permet une identification par rayonnement radiofréquence des objets porteurs d'une puce, le porteur de la montre devrait se trouver à proximité d'un interrogateur. Pour transmettre des informations à l'interrogateur, une étiquette RFID est généralement munie d'une puce électronique associée à une antenne.

Les moyens d'actionnement d'une alerte sont enclenchés lorsque l'unité de contrôle indique qu'au moins un composant intégré dans la boite de montre nécessite une intervention extérieure pour le bon fonctionnement de la montre, notamment un étalonnage du capteur ou un changement de la source d'alimentation.

Comme illustré à la figure 7, l'intérieur d'une boite de montre 13 permet de voir le logement 12, représenté schématiquement, dans lequel est disposé le capteur 11, le capteur 11 étant connecté au microprocesseur 17 contenant notamment une mémoire interne.

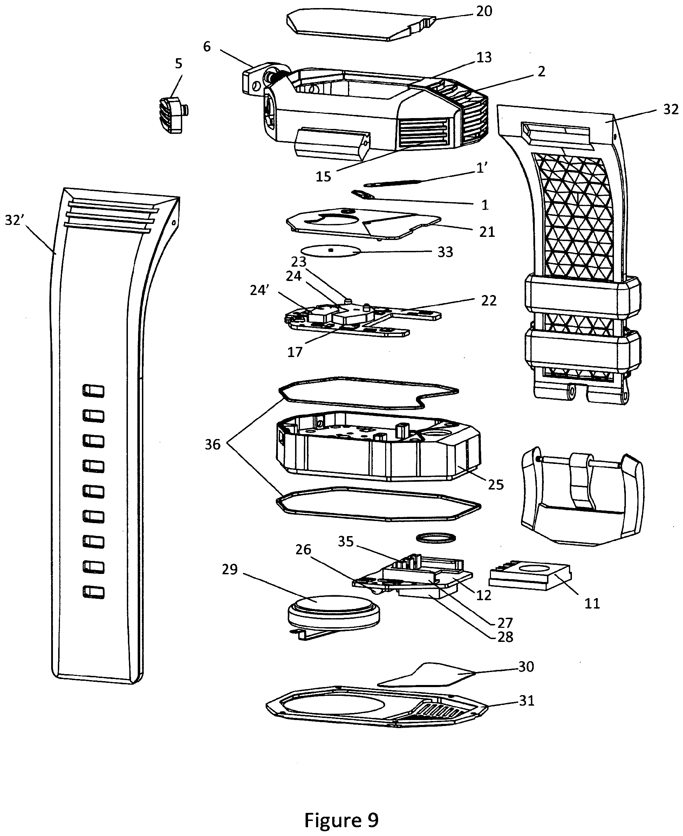

La montre comporte un dispositif d'étanchéité conventionnel. Par exemple, dans la montre illustrée aux figures 8 et 9, la boite de montre 13 comporte un container 25 pris en sandwich entre deux joints 36 et un support de capteur 27 venant se connecter au moyen de connecteurs 35 avec le container 25. Une glace 20 et un fond 31 ferment la boite de montre 13, le fond 31 comportant une grille pour laisser passer un son émis par un buzzer 28, une feuille 30 assurant l'étanchéité entre la grille du fond 31 et une partie électrique du dispositif comportant notamment le buzzer 28.

Comme illustré aux figures 8 et 9, une montre-bracelet selon la présente invention, comporte deux boutons poussoirs 5, 6 et une led 23. La montre comporte un cadran 21 dont une première zone de cadran qui comporte un disque 33 affichant différentes grandeurs ou des modes de fonctionnement et une deuxième zone de cadran qui comporte un affichage de la mesure de chaque grandeur sur deux échelles 4, 4' au moyen d'aiguilles 1, 1' qui indiquent également l'heure courante.

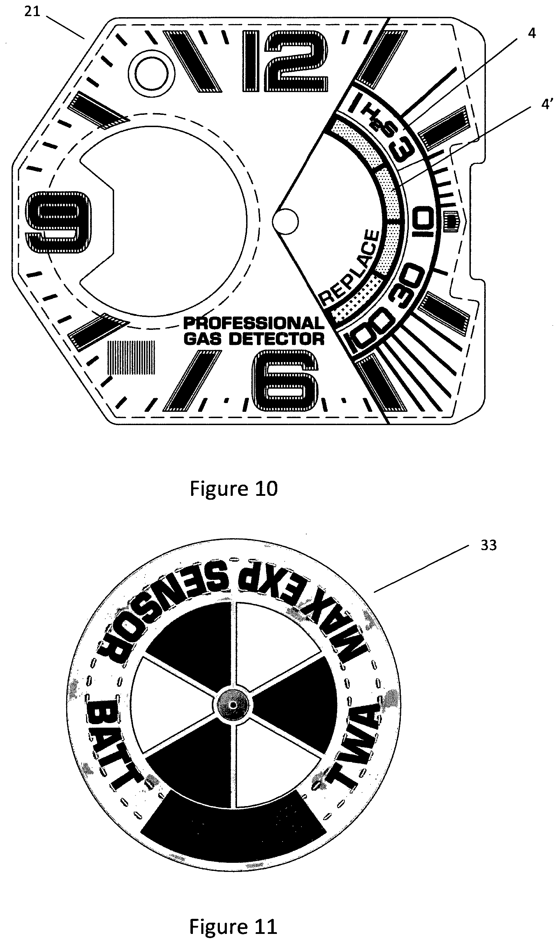

Comme illustré à la figure 11, le disque 33 comporte des inscriptions qui correspondent à des grandeurs à mesurer. Ces inscriptions sont écrites de manière circonférentielle sur le disque 33 et on peut, dans cet exemple distinguer les inscriptions DETECT, TWA, MAX EXP, SENSOR et BATT.

L'inscription DETECT correspond à l'index « 0 » du disque 33 en mode trotteuse lorsque la montre est en fonctionnement normal ou à un mode Alarme lorsque la montre a détecté la présence d'un gaz. L'inscription TWA (Time Weighted Average) correspond à la moyenne pondérée dans le temps des expositions au gaz. L'inscription MAX EXP correspond à la valeur mesurée maximale de la concentration de gaz. L'inscription SENSOR correspond à la durée de vie du capteur. L'inscription BATT correspond à la durée de vie de la batterie. D'autres inscriptions peuvent être disposées sur le disque 33 comme par exemple une inscription ALARM qui correspondrait à indiquer la présence d'un gaz.

La figure 10 permet de mieux visualiser le cadran 21 et notamment les deux zones de cadran, la première qui comporte une ouverture pour agencer le disque 33 et la deuxième qui comporte l'affichage de la mesure de chaque grandeur sur les deux échelles 4, 4' au moyen des aiguilles 1, 1' qui indiquent également, dans l'état ou le mode « heure », l'heure courante. Les échelles 4, 4' ont une forme arquée. Les deux échelles 4, 4' sont concentriques autour d'un centre qui correspond à l'axe autour duquel tourne les aiguilles 1, 1'. La première échelle 4, la plus externe par rapport à l'axe de rotation des aiguilles 1, 1' indique la concentration de gaz, ici sur une échelle de 0 à 100. L'échelle 4 s'étend sur environ 120°, environ 30° étant destinés à indiquer la nature du gaz, le restant étant réservé pour agencer une graduation ou une indication des valeurs. La deuxième échelle 4', la plus proche de l'axe de rotation des aiguilles 1, 1' indique la durée de vie du capteur ou de la batterie. L'échelle 4' de durée de vie s'étend sur environ 120°.

Comme illustré à la figure 8, le centre de rotation du disque 33 et l'axe de rotation des aiguilles 1, 1' sont alignés de manière avec la valeur affichée représentant la moitié de la durée de vie. L'aiguille des heures 1 indique les informations sur la deuxième échelle interne 4' et l'aiguille des minutes 1' indique les informations sur la première échelle 4. L'aguille des heures 1 est plus large que l'aiguille des minutes 1' d'environ entre 10% et 30%, de préférence 12%. L'aiguille des minutes 1' comporte une partie pleine et une partie ajourée. La partie ajourée permet de pouvoir lire l'information positionnée sur la première échelle 4. L'aiguille des minutes 1' est plus longue que l'aiguille des heures 1 de telle sorte que la longueur de l'aiguille des heures 1 correspond environ à la partie pleine de l'aiguille des minutes 1' de manière à toujours pouvoir lire l'information sur la première échelle 4. De préférence, l'aiguille des heures 1 est plus courte que l'aiguille des minutes 1' d'environ 20% à 30%, typiquement 25%.

La montre telle qu'illustrée à la figure 8 comporte un affichage mécanique à base d'aiguilles 1, 1' et d'un disque 33, le déplacement des aiguilles 1, 1' et du disque 33 étant liés les uns par rapport aux autres en fonction des modes activés par un utilisateur de la montre.

La montre comporte un mode Sommeil, un mode Alarme et un mode Heure. Le mode Sommeil permet de désactiver le capteur de gaz 11. En général, la montre est livrée à un utilisateur en mode sommeil et, dans ce cas, le capteur de gaz 11 est désactivé, les aiguilles 1, 1' sont parquées immobiles à 9h et le disque 33 est parqué immobile sur une indication SENSOR.

Dans cet exemple, la montre mesure en permanence la présence de gaz dans l'air ambiant. En cas de détection de gaz, la montre passe automatiquement en mode Alarme. En mode Alarme, le disque 33 indique DETECT. L'aiguille des minutes 1' affiche la concentration actuelle du gaz sur la première échelle 4 de concentration de gaz. L'aiguille des heures 1 affiche la concentration de gaz maximale atteinte. Lorsque le gaz n'est plus détecté, la montre passe automatiquement en mode Heure et l'heure courante est affichée.

En plus de mesurer en permanence la présence de gaz, la montre peut afficher d'autres grandeurs qu'il convient de sélectionner au préalable grâce au disque 33. Par exemple, il est possible de mesurer l'état de la batterie 29, l'état du capteur de gaz 11, la moyenne pondérée dans le temps des expositions au gaz et la valeur maximale mesurée de la concentration de gaz.

La montre bracelet comporte également un mode réglage, indiqué sur le disque 33 par l'indication Detect pendant lequel un réglage de l'heure ou un étalonnage du capteur 11 peut être réalisé.

Dans ce mode réglage, pour activer la montre, il faut presser simultanément sur les deux poussoirs 5, 6 pendant un temps donné, cinq secondes par exemple. Le capteur de gaz 11 est activé et la montre bracelet passe en mode Heure.

En mode Heure, le mode de fonctionnement normal de la montre, les aiguilles 1, 1' indiquent l'heure courante, le disque 33 tourne à la vitesse d'une trotteuse, soit un tour par minute, l'index 0 est le texte DETECT dans un guichet 34.

Comme expliqué précédemment, la montre mesure en permanence la présence de gaz dans l'air ambiant. En cas de détection de gaz, la montre passe automatiquement en mode Alarme. Lorsque le gaz n'est plus détecté, la montre passe automatiquement en mode Heure. En mode Alarme, l'aiguille des minutes 1' affiche la concentration actuelle du gaz sur l'échelle 4 de concentration de gaz. L'aiguille des heures 1 affiche la concentration maximale atteinte sur l'échelle 4 de concentration de gaz. En mode alarme, un buzzer 28 peut émettre un signal sonore, un vibreur 26 peut s'enclencher à différents intervalles, et une Led 23 peut s'allumer de manière fixe ou en clignotant.

Dans cet exemple, il existe quatre niveaux d'alarme selon la concentration de gaz dans l'air mesurée par le capteur 11, chaque niveau d'alarme émettant un signal sonore différent, par exemple 1 bip par minute pour une concentration de 1ppm (partie par million ou mg.L-1 ), 2 bips par minutes pour une concentration de 5 ppm, 30 bips par minute pour une concentration de 10 ppm et 60 bips par minute pour une concentration de 15 ppm.

Lorsque la montre est dans le mode Alarme, il est tout de même possible de consulter l'heure en appuyant le bouton poussoir 5. La montre indique l'heure et retourne automatiquement en mode Alarme après un temps donné, par exemple 10 secondes.

Dans cet exemple, lorsque la montre a été exposée à un gaz, il est possible de consulter les deux valeurs suivantes :
- TWA (Time Weighted Average), c'est la moyenne pondérée dans le temps des expositions au gaz.
- Exposition maximale, c'est la valeur mesurée maximale de la concentration de gaz.

Il est possible de consulter la TWA et l'Exposition Maximale uniquement lorsque la montre ne se trouve pas exposée à un gaz.

Pour afficher la valeur du TWA, depuis le mode Heure, il faut appuyer sur le poussoir 6 pour afficher la TWA. Le disque 33 indique TWA. Les deux aiguilles 1, 1' se superposent et indiquent la TWA sur l'échelle 4 de concentration de gaz. Si la montre n'a pas été exposée à un gaz, ou si la TWA et inférieure à 1 ppm, les aiguilles 1, 1' se positionnent sur 12h.

Un appui simultané sur les deux poussoirs 5, 6 pendant un temps donné, par exemple 3 secondes réinitialise la TWA. Depuis le mode TWA, la montre retourne automatiquement en mode Heure après un temps donné, par exemple 10 secondes.

Pour afficher la valeur maximale d'exposition à un gaz, depuis le mode TWA, il faut appuyer sur le poussoir 6. Le disque 33 indique MAX EXP à travers le guichet 34. Les deux aiguilles 1, 1' se superposent et indiquent l'exposition maximale sur l'échelle 4 de concentration de gaz. Si la montre n'a pas été exposée à un gaz, les aiguilles 1, 1' se positionnent sur 12h.

Un appui simultané sur les deux poussoirs 5, 6 pendant un temps donné, par exemple 3 secondes réinitialise l'Exposition Maximale. Depuis le mode Exposition Maximale, la montre retourne automatiquement en mode Heure après un temps donné, par exemple 10 secondes.

Dans l'exemple illustré aux figures 8 et 9, deux grandeurs essentielles peuvent être mesurées pour connaître leur état de fonctionnement, en l'occurrence, le capteur 11 et la pile 29. En effet, il est important de connaître l'état de vieillissement du capteur 11 dont la durée de vie se compte généralement en année et la durée de vie de la pile 29 dont la durée de vie se compte également en année.

Le capteur 11 a une durée de vie limitée dans le temps. Après cette période, il est nécessaire de le changer. Comme illustré notamment à la figure 9, le capteur 11 vient s'agencer dans un support 27 à la manière d'un tiroir, en coulissant dans le support 27 jusqu'à se contacter avec un connecteur 35 qui permet une connexion électrique avec une carte électronique 22. Cette carte électronique 22 comporte notamment le microcontroleur 17, et deux moteurs 24, 24' agencés pour entraîner notamment les aiguilles 1, 1' et le disque 33.

Le capteur 11 ayant une durée de vie limitée, il doit donc être changé. Après avoir changé le capteur 11, il faut réinitialiser la montre. Il est possible de consulter la durée de vie du capteur 11 et la durée de vie de la pile 29 uniquement lorsque la montre ne se trouve pas exposée à un gaz.

Pour afficher la durée de vie du capteur 11, depuis le mode Heure, il faut appuyer sur le poussoir 5 pour afficher SENSOR sur le disque 33. Les deux aiguilles 1, 1' s'alignent en opposé et l'aiguille des heures 1 indique le vieillissement du capteur 11 sur une deuxième échelle 4'. Depuis le mode SENSOR, la montre retourne automatiquement en mode Heure après un temps donné, par exemple 10 secondes.

Lorsque le capteur 11 arrive en fin de vie, la montre n'affiche plus l'heure mais le disque 33 indique SENSOR et l'aiguille des heures 1 indique le point le plus bas de la deuxième échelle 4' (figure 10).

Pour afficher la durée de vie de la pile 29, depuis le mode SENSOR sur le disque 33, il faut appuyer le poussoir 5 pour afficher BATT (pour Battery) sur le disque 33. Les deux aiguilles 1, 1' s'alignent en opposé et l'aiguille des heures 1 indique l'état de batterie sur la seconde échelle 4'.

Depuis le mode BATT, la montre retourne automatiquement en mode Heure après un temps donné, par exemple 10 secondes.

Lorsque la montre subit un choc, il est possible que le disque 33 ou les aiguilles 1, 1' perdent leur alignement. Il est alors nécessaire de les calibrer.

Pour la calibration des aiguilles 1, 1' et du disque 33, depuis le mode Heure, il faut appuyer simultanément sur les deux poussoirs 5, 6. Un premier bip indique que la montre passe en mode Réglage de l'heure.

Tout en maintenant la pression sur les deux poussoirs 5, 6, après un temps défini, par exemple 3 secondes, un deuxième bip indique que la montre passe en mode Calibration. Le disque 33 indique DETECT et les aiguilles 1, 1' se superposent sur 12h. Si le disque 33 n'est pas précisément sur DETECT ou si les aiguilles 1, 1' ne sont pas précisément sur 12h, une calibration est nécessaire.

Il faut alors appuyer sur le poussoir 6 pour tourner l'aiguille des heures 1 d'un pas dans le sens horaire ou il faut appuyer sur le poussoir 5 pour tourner l'aiguille des heures 1 d'un pas dans le sens antihoraire jusqu'à alignement de l'aiguille des heures 1 précisément sur 12h. Il faut procéder de la même manière pour calibrer l'aiguille des minutes 1'.

Ensuite, il faut appuyer simultanément sur les deux poussoirs 5, 6 pour calibrer le disque 33. Il faut appuyer sur le poussoir 6 pour tourner le disque 33 d'un pas dans le sens horaire ou appuyer sur le poussoir 5 pour tourner le disque 33 d'un pas dans le sens antihoraire jusqu'à alignement précis du disque 33 sur l'inscription DETECT à travers le guichet 34. Un appui simultané sur les deux poussoirs 5, 6 termine la calibration.

Sans action, la montre retourne automatiquement en mode Heure après un temps donné, par exemple 30 secondes.

Comme expliqué précédemment, lorsque la montre n'est pas utilisée pour une longue période, ou par exemple lorsqu'elle est envoyée pour une maintenance, il est possible de la mettre en mode Sommeil.

En mode Sommeil, le capteur de gaz 11 est désactivé, les aiguilles 1, 1' sont parquées immobiles à 9h et le disque 33 est parqué immobile sur SENSOR. L'heure est conservée par la montre.

Pour un passage de la montre en mode Sommeil, depuis le mode Heure, il faut appuyer simultanément sur les deux poussoirs 5, 6. Un premier bip indique que la montre passe en mode Réglage de l'heure. Le disque 33 indique DETECT.

Sans relâcher les deux poussoirs 5, 6, après un temps donné, par exemple 3 secondes un deuxième bip indique que la montre passe en mode Calibration, les aiguilles 1, 1' se superposent alors sur 12h.

Toujours sans relâcher les deux poussoirs 5, 6, après un temps donné, par exemple 5 secondes, un troisième bip indique que la montre passe en mode Sommeil. Le disque 33 indique SENSOR. Les deux aiguilles 1, 1' se superposent alors sur 9h.

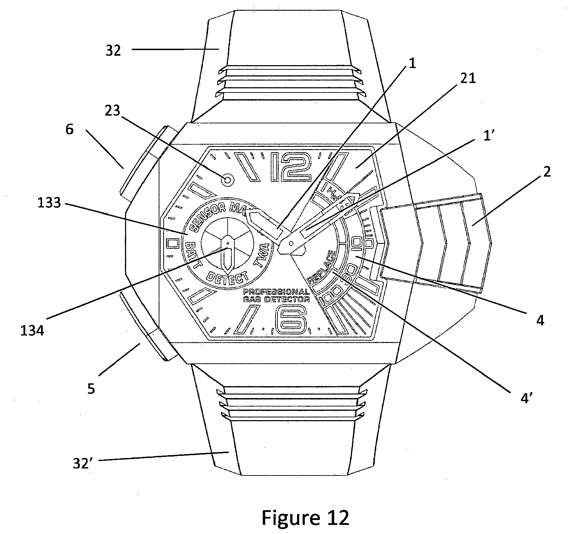

Dans une autre version illustrée notamment à la figure 12, une montre-bracelet selon la présente invention, comporte deux boutons poussoirs 5, 6 et une led 23. La montre comporte un cadran 21 dont une première zone de cadran qui comporte un premier affichage à aiguille 133 affichant différentes grandeurs ou des modes de fonctionnement au moyen d'une aiguille 134 et une deuxième zone de cadran qui comporte un affichage de la mesure de chaque grandeur sur deux échelles 4, 4' au moyen d'aiguilles 1, 1' qui, dans le mode « heure » indiquent également l'heure courante comme illustrée à la figure 12. A la différence de l'exemple de la figure 8 précédemment décrit, le cadran 21 et l'affichage à aiguilles 133 ne font qu'un. Ainsi, l'aiguille 134 tourne à la vitesse d'une trotteuse en mode « Heure », soit un tour par minute. A la demande de l'utilisateur, par exemple pour les mesures de durée de vie de batterie ou de capteur, ou de manière automatique, par exemple à la détection d'un gaz, l'aiguille 134 pointe sur le mode/état correspondant sur le premier affichage à aiguille 134.

La montre de la présente invention permet donc de détecter un gaz dans une zone. Cependant, lorsque les moyens d'alerte sont enclenchés, il se peut que le porteur de la montre ne puisse plus se déplacer car il se serait par exemple évanoui. De ce fait, la montre comporte non seulement des moyens de communications sans fil, mais également des moyens de localisation. En effet, il est très important pour les personnes qui ont reçu une alerte de pouvoir localiser le porteur de la montre. La montre peut donc comporter un capteur de pression 40 grâce auquel la montre pourra connaître son altitude par rapport au niveau de la mer avec un très haut niveau de précision. Ainsi, le porteur de la montre équipée de ce capteur de pression 40 pourra être retrouvé précisément, par exemple à quel étage il se situe s'il se trouve dans un bâtiment à étages.

Dans une autre version de la montre, un simple capteur de géolocalisation peut suffire à retrouver la zone où se trouve le porteur de la montre sans pour autant en définir l'étage s'il est dans un bâtiment à étages.

La montre de la présente invention est un équipement de protection individuelle pouvant détecter un plusieurs gaz permettant de sécuriser une zone dans laquelle se trouve son utilisateur.

## Revendications

1. Dispositif pour mesurer la concentration d'un gaz composant l'air ambiant, le dispositif comportant une montre-bracelet comprenant :
- Une boite de montre (13) contenant un mouvement horloger, des organes indicateurs de l'heure (1, 1', 3, 9) visibles à travers une glace de la boite de montre (13),
- Au moins un capteur (11) agencé pour mesurer la concentration d'un gaz composant de l'air ambiant, ce capteur (11) étant situé dans un logement (12) de la boite de montre (13) ou d'un élément rapporté (14) à la boite de montre et présentant une surface à l'intérieur de ce logement (12) exposé à l'air ambiant à travers au moins un orifice (2), l'orifice (2) se trouvant sur une partie de la boite de montre (13) ou sur l'élément rapporté (14) qui n'est pas en contact avec le poignet du porteur lorsque la montre-bracelet est porté au poignet,
- Un organe (4, 4', 8, 9, 10) indicateur de la valeur mesurée de la concentration de gaz, notamment une échelle graduée sur un cadran, et
- Des moyens pour actionner une alerte ;
Le dispositif comportant deux états de fonctionnement, un état « heure » et un état « alarme »,
**Caractérisé en ce que**
le dispositif est configuré pour fonctionner selon une séquence opérationnelle complète comprenant les trois étapes suivantes a), b) et c) :
a) En l'état « heure » les organes indicateurs de l'heure (1, 1', 3, 9) indiquent l'heure et le capteur (11) mesure en permanence la concentration de gaz sans l'afficher et sans actionner une alerte en l'absence d'une concentration mesurée de gaz ;
b) Au cas où le capteur (11) détecte une concentration d'un gaz lorsque le capteur est dans l'état « heure », le dispositif bascule automatiquement en l'état « alerte » dans lequel les organes indicateurs de l'heure (1, 1', 3, 9) sont activés pour indiquer l'heure ou inactivés pour ne pas indiquer l'heure et dans lequel l'organe indicateur de la valeur mesurée de la concentration de gaz (4, 4', 8, 9, 10) affiche cette valeur mesurée, et en outre les moyens pour actionner une alerte actionnent une alerte lors de l'atteinte ou du franchissement d'un seuil de la concentration de gaz mesurée par le capteur (11) ; et
c) Au cas où le capteur (11) ne détecte plus une concentration d'un gaz lorsque le capteur est dans l'état « alerte », le dispositif bascule automatiquement en l'état « heure » où les organes indicateurs de l'heure (1, 1', 3, 9) indiquent l'heure et le capteur (11) mesure en permanence la concentration de gaz sans l'afficher et sans déclencher d'alerte en l'absence de concentration de gaz mesurée ;
et dans lequel la montre-bracelet comprend en outre un moyen de communication sans fil pour transmettre des informations à un autre appareil, à savoir le moyen de communication sans fil est agencé pour transmettre à un smartphone ou à un ordinateur, des informations indiquant que ladite valeur seuil de la concentration de gaz mesurée dans l'air ambiant a été atteinte ou dépassée.

2. Dispositif selon la revendication 1, dans lequel les organes indicateurs de l'heure sont, à l'exception de ce qui est spécifié ici, normalement non-opérationnels pendant que le dispositif est dans l'état « alerte », le dispositif comportant un bouton de commande, actionnable lorsque le dispositif en dans l'état « alerte », pour que ces organes viennent indiquer l'heure pendant une durée prédéfinie et ensuite le dispositif retourne en état « alerte ».

3. Dispositif selon l'une des revendications 1 ou 2, où la montre-bracelet comporte un moyen d'alerte, notamment une alarme lumineuse (23) et/ou une alarme sonore (28) et/ou une alarme vibrante (26).

4. Dispositif selon l'une des revendications précédentes, dans lequel la montre-bracelet comporte:
- une unité de contrôle agencée pour mesurer l'état de fonctionnement et/ou la durée de vie de composants intégrés dans la boite de montre, notamment le capteur (11) ou la durée de vie d'une source d'alimentation (29) ; et/ou
- une unité de stockage agencée pour conserver un historique des valeurs mesurées de la concentration de gaz et optionnellement les mesures des composants intégrés dans la boite de montre et inspectés par une unité de contrôle.

5. Dispositif selon la revendication 4, dans lequel lesdits moyens pour actionner une alerte sont enclenchés lorsque l'unité de contrôle indique qu'au moins un composant intégré dans la boite de montre nécessite une intervention extérieure.

6. Dispositif selon l'une des revendications précédentes, comportant :
- un jeu d'aiguilles (1, 1', 3) pour afficher d'une part l'heure et d'autre part la valeur mesurée de la concentration de gaz, ou deux jeux d'aiguilles (1, 3, 18) pour afficher séparément ces fonctions ; ou
- un affichage digital (9) pour afficher d'une part l'heure et d'autre part pour afficher la valeur mesurée de la concentration de gaz, ou deux affichages digitaux (9, 10) pour afficher séparément ces fonctions ; ou
- un jeu d'aiguilles (1, 3) pour l'affichage de l'heure et un affichage digital (10) pour afficher la valeur mesurée de la concentration de gaz mesurée ou vice et versa.

7. Dispositif selon l'une des revendications précédentes, dans lequel la montre-bracelet comporte une unité de traitement apte à recevoir des informations d'un appareil externe pour configurer la montre, notamment pour étalonner le capteur (11) ou pour la mise à jour de l'heure.

8. Dispositif selon l'une des revendications précédentes, comportant une unité de traitement et/ou une unité de contrôle et/ou une unité de stockage dans un seul ou plusieurs microprocesseurs programmés (17).

9. Dispositif selon l'une des revendications précédentes, dans lequel la montre-bracelet comporte une pluralité de capteurs (11) agencés chacun pour mesurer respectivement la concentration d'un gaz particulier composant de l'air ambiant.

10. Dispositif selon l'une des revendications précédentes, dans lequel la montre-bracelet comporte au moins un capteur (11) apte à mesurer du sulfure d'hydrogène, ou du monoxyde de carbone.

11. Dispositif selon l'une des revendications précédentes, dans lequel un capteur de géolocalisation (40) est agencé pour localiser le dispositif dans une position à deux dimensions et optionnellement à trois dimensions.

12. Dispositif selon l'une des revendications précédentes, dans lequel le dispositif comporte également un état « sommeil » dans lequel le capteur (11) est désactivé, et au moins un bouton poussoir, actionnable lorsque le dispositif en dans l'état « sommeil » pour activer le dispositif en état « heure ».

13. Dispositif selon l'une des revendications précédentes, dans lequel les moyens pour actionner une alerte actionnent une alerte à plusieurs niveaux selon la concentration de gaz dans l'air ambiant mesurée par le capteur, chaque niveau d'alerte correspondant à un ou plusieurs bips sonores ou lumineuse ou des vibrations, dont l'intensité et la fréquence peuvent varier.

14. Dispositif selon l'une des revendications précédentes, dans lequel les organes indicateurs de l'heure comportent un cadran et au moins deux aiguilles (1, 1') de longueurs différentes pivotantes autour d'une axe ;
- Le dispositif comprenant en outre un segment du cadran s'étendant au plus sur 150° et de préférence au plus sur 120°, ce segment de cadran portant vers sa périphérie au moins deux zones arquées avec des graduations ou autres indications concernant une concentration de gaz mesurée et/ou une opération de maintenance du dispositif, ces zones étant décalées radialement ;
- Les aiguilles coopérant avec le cadran pour indiquer l'heure lors de l'état « heure » ; et
- Les aiguilles de longueur différentes coopèrent avec les zones arquées décalées radialement lors de l'état « alerte » pour donner au moins une indication relative à la concentration de gaz mesurée et facultativement une indication de maintenance du dispositif.

15. Dispositif selon l'une des revendications précédentes, comportant un cadran avec un disque dont la circonférence porte une série d'indications relative à la concentration du gaz mesurée, le fonctionnement du dispositif et/ou la durée de vie de composants intégrés dans la boîte de montre et/ou la durée de vie d'une source d'alimentation;
- Le dispositif comportant en outre des moyens pour faire tourner le disque ou pour faire tourner un organe indicateur par rapport au disque afin d'indiquer une indication choisie et, selon l'indication choisie, pour faire fonctionner le dispositif ou pour permettre de calculer différents paramètres de concentration de gaz ou faire les opérations nécessaires pour remettre le dispositif en état de fonctionnement.

## Patentansprüche

1. Vorrichtung zum Messen der Konzentration eines Gases, das ein Bestandteil der Umgebungsluft ist, wobei die Vorrichtung eine Armbanduhr aufweist, umfassend:
- ein Uhrengehäuse (13), das ein Uhrwerk, Uhrzeitangabeorgane (1, 1', 3, 9), die durch ein Glas des Uhrengehäuses (13) sichtbar sind, enthält,
- mindestens einen Sensor (11), der angeordnet ist, um die Konzentration eines Gases, das Bestandteil der Umgebungsluft ist, zu messen, wobei dieser Sensor (11) sich in einer Aufnahme (12) des Uhrengehäuses (13) oder eines an dem Uhrengehäuse angebrachten Elements (14) befindet und eine Oberfläche im Inneren dieser Aufnahme (12) vorweist, die durch mindestens eine Öffnung (2) der Umgebungsluft ausgesetzt ist, wobei die Öffnung (2) sich an einem Teil des Uhrengehäuses (13) oder an dem angebrachten Element (14) befindet, das nicht mit dem Handgelenk des Trägers in Berührung kommt, wenn die Armbanduhr an dem Handgelenk getragen wird,
- ein Organ (4, 4', 8, 9, 10), das den gemessenen Wert der Gaskonzentration angibt, insbesondere eine Skala auf einem Zifferblatt, und
- Mittel zum Auslösen eines Alarms;
wobei die Vorrichtung zwei Betriebszustände, einen "Uhrzeit"-Zustand und einen "Alarm"-Zustand, aufweist,
**dadurch gekennzeichnet, dass**
die Vorrichtung konfiguriert ist, um in einer vollständigen Betriebssequenz zu arbeiten, umfassend die folgenden drei Schritte a), b) und c):
a) in dem "Uhrzeit"-Zustand geben die Uhrzeitangabeorgane (1, 1', 3, 9) die Uhrzeit an und der Sensor (11) misst ständig die Gaskonzentration, ohne diese anzuzeigen und ohne einen Alarm auszulösen, bei Abwesenheit einer gemessenen Gaskonzentration;
b) falls der Sensor (11) eine Gaskonzentration erfasst, wenn der Sensor sich in dem "Uhrzeit"-Zustand befindet, wechselt die Vorrichtung automatisch in den "Alarm"-Zustand, in dem die Uhrzeitangabeorgane (1, 1', 3, 9) aktiviert, um die Uhrzeit anzugeben, oder inaktiviert sind, um die Uhrzeit nicht anzugeben, und in dem das Angabeorgan für den gemessenen Wert der Gaskonzentration (4, 4', 8, 9, 10) diesen gemessenen Wert anzeigt, und ferner die Mittel zum Auslösen eines Alarms einen Alarm auslösen, wenn ein Schwellenwert der Gaskonzentration, die von dem Sensor (11) gemessen wird, erreicht oder überschritten wird; und
c) falls der Sensor (11) keine Gaskonzentration mehr erfasst, wenn der Sensor sich in dem "Alarm"-Zustand befindet, wechselt die Vorrichtung automatisch in den "Uhrzeit"-Zustand, in dem die Uhrzeitangabeorgane (1, 1', 3, 9) die Uhrzeit angeben und der Sensor (11) ständig die Gaskonzentration misst, ohne diese anzuzeigen und ohne einen Alarm zu triggern, bei Abwesenheit der gemessenen Gaskonzentration;
und wobei die Armbanduhr ferner ein drahtloses Kommunikationsmittel zum Übertragen von Informationen an eine andere Einrichtung umfasst, wobei nämlich das drahtlose Kommunikationsmittel angeordnet ist, um Informationen, die angeben, dass der Schwellenwert der in der Umgebungsluft gemessenen Gaskonzentration erreicht oder überschritten wurde, an ein Smartphone oder an einen Computer zu übertragen.

2. Vorrichtung nach Anspruch 1, wobei die Uhrzeitangabeorgane, mit Ausnahme der hier angegebenen, normalerweise nicht betriebsbereit sind, während die Vorrichtung sich in dem "Alarm"-Zustand befindet, wobei die Vorrichtung einen Bedienungsknopf aufweist, der betätigt werden kann, wenn die Vorrichtung sich in dem "Alarm"-Zustand befindet, damit diese Organe die Uhrzeit während einer vordefinierten Zeitdauer angeben und die Vorrichtung dann in den "Alarm"-Zustand zurückkehrt.

3. Vorrichtung nach einem der Ansprüche 1 oder 2, wobei die Armbanduhr ein Alarmmittel, insbesondere einen leuchtenden Alarm (23) und/oder einen akustischen Alarm (28) und/oder einen Vibrationsalarm (26), aufweist.

4. Vorrichtung nach einem der vorstehenden Ansprüche, wobei die Armbanduhr aufweist:
- eine Prüfeinheit, die angeordnet ist, um den Betriebszustand und/oder die Lebensdauer von in das Uhrengehäuse integrierten Bestandteilen, insbesondere des Sensors (11), oder die Lebensdauer einer Stromquelle (29) zu messen; und/oder
- eine Speichereinheit, die angeordnet ist, um eine Historie der gemessenen Werte der Gaskonzentration und optional der von einer Prüfeinheit kontrollierten Messungen von den in das Uhrengehäuse integrierten Bestandteilen zu speichern.

5. Vorrichtung nach Anspruch 4, wobei die Mittel zum Auslösen eines Alarms eingeschaltet werden, wenn die Prüfeinheit angibt, dass mindestens ein in das Uhrengehäuse integrierter Bestandteil einen externen Eingriff erfordert.

6. Vorrichtung nach einem der vorstehenden Ansprüche, die aufweist:
- einen Satz von Zeigern (1, 1', 3), um einerseits die Uhrzeit und andererseits den gemessenen Wert der Gaskonzentration anzuzeigen, oder zwei Sätze von Zeigern (1, 3, 18), um diese Funktionen getrennt anzuzeigen; oder
- eine Digitalanzeige (9), um einerseits die Uhrzeit und andererseits den gemessenen Wert der Gaskonzentration anzuzeigen, oder zwei Digitalanzeigen (9, 10), um diese Funktionen getrennt anzuzeigen; oder
- einen Satz von Zeigern (1, 3), um die Uhrzeit anzuzeigen, und eine Digitalanzeige (10), um den gemessenen Wert der gemessenen Gaskonzentration anzuzeigen, oder umgekehrt.

7. Vorrichtung nach einem der vorstehenden Ansprüche, wobei die Armbanduhr eine Verarbeitungseinheit, die geeignet ist, Informationen von einer externen Einrichtung zu empfangen, um die Uhr zu konfigurieren, insbesondere um den Sensor (11) zu kalibrieren oder die Uhrzeit zu aktualisieren, aufweist.

8. Vorrichtung nach einem der vorstehenden Ansprüche, die eine Verarbeitungseinheit und/oder eine Prüfeinheit und/oder eine Speichereinheit in einem einzigen oder mehreren programmierten Mikroprozessoren (17) aufweist.

9. Vorrichtung nach einem der vorstehenden Ansprüche, wobei die Armbanduhr eine Vielzahl von Sensoren (11), die jeweils angeordnet sind, um die Konzentration eines bestimmten Gases, das Bestandteil der Umgebungsluft ist, jeweils zu messen, aufweist.

10. Vorrichtung nach einem der vorstehenden Ansprüche, wobei die Armbanduhr mindestens einen Sensor (11), der geeignet ist, Schwefelwasserstoff oder Kohlenmonoxid zu messen, aufweist.

11. Vorrichtung nach einem der vorstehenden Ansprüche, wobei ein Geolokalisierungssensor (40) angeordnet ist, um die Vorrichtung in einer zweidimensionalen und optional dreidimensionalen Position zu lokalisieren.

12. Vorrichtung nach einem der vorstehenden Ansprüche, wobei die Vorrichtung auch einen "Schlaf'-Zustand, in dem der Sensor (11) deaktiviert ist, und mindestens einen Druckknopf aufweist, der betätigbar ist, wenn die Vorrichtung sich in dem "Schlaf'-Zustand befindet, um die Vorrichtung in den "Uhrzeit"-Zustand zu aktivieren.

13. Vorrichtung nach einem der vorstehenden Ansprüche, wobei die Mittel zum Auslösen eines Alarms einen mehrstufigen Alarm gemäß der von dem Sensor gemessenen Gaskonzentration in der Umgebungsluft auslösen, wobei jede Alarmstufe einem oder mehreren akustischen oder leuchtenden Tonzeichen oder Vibrationen entspricht, deren Intensität und Frequenz variieren können.

14. Vorrichtung nach einem der vorstehenden Ansprüche, wobei die Uhrzeitangabeorgane ein Zifferblatt und mindestens zwei Zeiger (1, 1') unterschiedlicher Länge, die um eine Achse schwenkbar sind, aufweist;
- die Vorrichtung ferner umfassend ein Segment des Zifferblatts, das sich höchstens über 150° und vorzugsweise höchstens über 120° erstreckt, wobei dieses Zifferblattsegment zu seinem Umfang hin mindestens zwei bogenförmige Bereiche mit Gradeinteilungen oder anderen Angaben trägt, die sich auf eine gemessene Gaskonzentration und/oder einen Wartungsvorgang der Vorrichtung beziehen, wobei diese Bereiche radial versetzt sind;
- die Zeiger, die mit dem Zifferblatt zusammenwirken, um in dem "Uhrzeit"-Zustand die Uhrzeit anzugeben; und
- die Zeiger unterschiedlicher Länge, die mit den radial versetzten bogenförmigen Bereichen in dem "Alarm"-Zustand zusammenwirken, um mindestens einen Hinweis bezogen auf die gemessene Gaskonzentration und beliebig einen Hinweis auf die Wartung der Vorrichtung zu geben.

15. Vorrichtung nach einem der vorstehenden Ansprüche, die ein Zifferblatt mit einer Scheibe, deren Umfang eine Reihe von Angaben bezogen auf die gemessene Gaskonzentration, den Betrieb der Vorrichtung und/oder die Lebensdauer der in das Uhrengehäuse integrierten Bestandteile und/oder die Lebensdauer einer Stromquelle trägt, aufweist;
- wobei die Vorrichtung ferner Mittel zum Bewegen der Scheibe oder zum Bewegen eines Angabeorgans relativ zu der Scheibe, um eine ausgewählte Angabe anzugeben, und, abhängig von der ausgewählten Angabe, zum Betreiben der Vorrichtung oder zum Ermöglichen eines Berechnens verschiedener Gaskonzentrationsparameter oder zum Durchführen notwendiger Schritte, um die Vorrichtung wieder in einen Betriebszustand zu versetzen, aufweist.

## Claims

1. A device for measuring the concentration of a gas composing the ambient air, the device comprising a wristwatch comprising:
- A watch case (13) containing a timepiece movement, time-indicating members (1, 1', 3, 9) visible through a glass of the watch case (13),
- At least one sensor (11) arranged to measure the concentration of a gas composing the ambient air, this sensor (11) being located in a housing (12) of the watch case (13) or an element (14) attached to the watch case and having a surface inside this housing (12) exposed to ambient air through at least one orifice (2), the orifice (2) being on a part of the watch case (13) or on the attached element (14) which is not in contact with the wearer's wrist when the wristwatch is worn on the wrist,
- A member (4, 4', 8, 9, 10) indicating the measured value of the gas concentration, in particular a graduated scale on a dial, and
- Means for actuating an alert;
The device comprising two operating states, a "time" state and an "alarm" state,
**Characterized in that**
the device is configured to operate according to a full operational sequence comprising the following three steps a), b) and c):
a) In the "time" step, the time-indicating members (1, 1', 3, 9) indicate the time, and the sensor (11) continuously measures the gas concentration without displaying it and without actuating an alert in the absence of a measured gas concentration;
b) If the sensor (11) detects a concentration of a gas when the sensor is in the "time" state, the device automatically switches to the "alert" state in which the time-indicating members (1, 1', 3, 9) are activated to indicate the time or inactivated to not indicate the time and wherein the member indicating the measured value of the gas concentration (4, 4', 8, 9, 10) displays this measured value, and furthermore the means for actuating an alert actuate an alert when reaching or crossing a threshold of the gas concentration measured by the sensor (11); and
c) If the sensor (11) is no longer detecting a concentration of a gas when the sensor is in the "alert" state, the device automatically switches to the "time" state where the time-indicating members (1, 1', 3, 9) indicate the time and the sensor (11) continuously measures the gas concentration without displaying it and without triggering an alert in the absence of measured gas concentration;
and wherein the wristwatch further comprises wireless communication means for transmitting information to another apparatus, namely the wireless communication means is arranged to transmit, to a smartphone or to a computer, information indicating that said threshold value of the measured gas concentration in the ambient air has been reached or exceeded.

2. The device according to claim 1, wherein the time-indicating members are, with the exception of what is specified here, normally non-operational while the device is in the "alert" state, the device comprising a control button, operable when the device in the "alert" state, so that these units indicate the time for a predefined duration and then the device returns to the "alert" state.

3. The device according to one of claims 1 or 2, wherein the wristwatch comprises an alert means, in particular a light alarm (23) and/or an audible alarm (28) and/or a vibrating alarm (26).

4. The device according one of the preceding claims, wherein the wristwatch comprises:
- a control unit arranged to measure the operating state and/or the lifetime of components integrated into the watch case, in particular the sensor (11) or the lifetime of a power source (29); and/or
- a storage unit arranged to maintain a history of the measured values of the gas concentration and optionally the measurements of the components integrated into the watch case and inspected by a control unit.

5. The device according to claim 4, wherein said means for actuating an alert are engaged when the control unit indicates that at least one component integrated into the watch case requires external intervention.

6. The device according to one of the preceding claims, comprising:
- a set of hands (1, 1', 3) for displaying on the one hand the time and on the other hand the measured value of the gas concentration, or two sets of hands (1, 3, 18) to display these functions separately; or
- a digital display (9) for displaying on the one hand the time and on the other hand for displaying the measured value of the gas concentration, or two digital displays (9, 10) to display these functions separately; or
- a set of hands (1, 3) for displaying the time and a digital display (10) for displaying the measured value of the measured gas concentration or vice versa.

7. The device according to one of the preceding claims, wherein the wristwatch comprises a processing unit capable of receiving information from an external apparatus for configuring the watch, in particular for calibrating the sensor (11) or for updating the time.

8. The device according to one of the preceding claims, comprising a processing unit and/or a control unit and/or a storage unit in one or more programmed microprocessors (17).

9. The device according to one of the preceding claims, wherein the wristwatch comprises a plurality of sensors (11) each arranged to respectively measure the concentration of a particular gas composing the ambient air.

10. The device according to one of the preceding claims, wherein the wristwatch comprises at least one sensor (11) capable of measuring hydrogen sulfide, or carbon monoxide.

11. The device according to one of the preceding claims, wherein a geolocation sensor (40) is arranged to locate the device in a two-dimensional and optionally three-dimensional position.

12. The device according to one of the preceding claims, wherein the device also comprises a "sleep" state wherein the sensor (11) is deactivated, and at least one push-button, operable when the device in the "sleep" state, to switch the device to the "time" state.

13. The device according to one of the preceding claims, wherein the means for actuating an alert actuate a multi-level alert depending on the concentration of gas in the ambient air measured by the sensor, each alert level corresponding to one or more sound or light beeps or vibrations, the intensity and frequency of which can vary.

14. The device according to one of the preceding claims, wherein the time-indicating members comprise a dial and at least two hands (1, 1') of different lengths pivoting about an axis;
- The device further comprising a segment of the dial extending at most over 150° and preferably at most over 120°, this dial segment bearing towards its periphery at least two arched areas with graduations or other indications regarding a measured gas concentration and/or a maintenance operation of the device, these areas being radially offset;
- The hands cooperating with the dial to indicate the time during the "time" state; and
- The hands of different length cooperate with the arched areas offset radially during the "alert" state to give at least one indication relating to the measured gas concentration and optionally a maintenance indication of the device.

15. The device according to one of the preceding claims, comprising a dial with a disc whose circumference carries a series of indications relating to the measured gas concentration, the operation of the device and/or the lifetime of components integrated into the watch case and/or the lifetime of a power source;
- The device further comprising means for rotating the disc or for rotating an indicator member relative to the disc in order to indicate a chosen indication and, according to the chosen indication, to operate the device or to make it possible to calculate various gas concentration parameters or perform the operations necessary to return the device to the operating state.
